# EUROPEAN PATENT SPECIFICATION

(11) **EP 1 753 297 B1**
(45) Date of publication and mention of the grant of the patent: **27.09.2017**
(21) Application number: 05735392.2
(22) Date of filing: 02.05.2005
(51) Int. Cl.: A23C 9/12, A23C 9/127, A23C 9/152, A23C 21/02

(54) **ENZYMATIC PROCESS FOR OBTAINING INCREASED YIELD OF LACTOBIONIC ACID**
ENZYMATISCHES VERFAHREN FÜR EINE ERHÖHTE LAKTOBIONSÄUREAUSBEUTE
PROCEDE ENZYMATIQUE POUR L'OBTENTION DE RENDEMENTS ACCRUS DE L'ACIDE LACTOBIONIQUE

(30) Priority: 03.05.2004 DK 200400702; 04.05.2004 US 568157 P; 24.06.2004 DK 200400983; 01.07.2004 US 584690 P
(43) Date of publication of application: 21.02.2007
(73) Proprietor: Novozymes A/S, 2880 Bagsværd (DK); Novozymes North America, Inc., Franklinton, North Carolina 27525 (US)
(72) Inventor: BUDTZ, Peter, DK-2000 Frederiksberg (DK); VINDELØV, Jannik Torben, DK-1905 Frederiksberg C (DK); NIELSEN, Per Munk, DK-3400 Hillerød (DK); ASHIE, Isaac, Raleigh, NC 27604 (US); NORDKVIST, Mikkel, DK-2400 Copenhagen NV (DK)
(74) Representative: Bauditz, Peter
(86) International application number: PCT/DK2005/000299
(87) International publication number: WO 2005/104859

(56) References cited:
- EP-A- 0 384 534
- WO-A-02/089592
- WO-A-03/037093
- US-A- 3 930 028
- US-A1- 2004 151 802
- MIYAMOTO ET AL.: "Production of lactobionic acid from whey by Pseudomonas sp. LS13-1" BIOTECHNOLOGY LETTERS, vol. 22, 2000, pages 427-430, XP002350037
- MURAKAMI H ET AL: "Fermentative production of lactobionic acid by Burkholderia cepacia." JOURNAL OF APPLIED GLYCOSCIENCE, vol. 50, no. 2, 2003, pages 117-120, XP008036393
- RAND A: "DIRECT ENZYMATIC CONVERSION OF LACTOSE TO ACID: GLUCOSE OXIDASE AND HEXOSE OXIDASE" JOURNAL OF FOOD SCIENCE, INSTITUTE OF FOOD TECHNOLOGISTS. CHICAGO, US, vol. 37, no. 5, 1972, pages 698-701, XP008000509 ISSN: 0022-1147
- WRIGHT D G ET AL: "DIRECT ENZYMATIC CONVERSION OF LACTOSE TO ACID: LACTOSE DEHYDROGENASE" JOURNAL OF FOOD SCIENCE, INSTITUTE OF FOOD TECHNOLOGISTS. CHICAGO, US, vol. 38, 1973, pages 1132-1135, XP008000518 ISSN: 0022-1147
- RAND A G ET AL: "DIRECT ENZYMATIC CONVERSION OF LACTOSE IN MILK TO ACID" 1 August 1975 (1975-08-01), JOURNAL OF DAIRY SCIENCE, AMERICAN DAIRY SCIENCE ASSOCIATION. CHAMPAIGN, ILLINOIS, US, PAGE(S) 1144-1150 , XP000644502 ISSN: 0022-0302 page 1145; figure 3

## Description

### FIELD OF THE INVENTION:

A process for obtaining increased yield and/or a reduced reaction time in enzymatic conversion of lactose to lactobionic acid comprising adding to a dairy substrate such as milk, whey or a lactose solution, a carbohydrate oxidase, capable of converting lactose to lactobionic acid, wherein pH of the process is controlled and maintained at a specified level.

### BACKGROUND OF THE INVENTION:

Lactose, commonly known as milk sugar, is the primary carbohydrate of milk. In milk based dairy products such as yoghurt and cheese, lactose may be considered a low value sugar due to e.g. lactose intolerance and due to its involvement in browning and crystallisation reactions. Lactose accounts for up to 75% of the total dry material in whey and accumulates in annual quantities of approximately 1.2 million tons worldwide without many profitable routes for the direct utilisation being available.

However, lactose may be converted to lactobionic acid (4-O-β-D-galactopyranosyl-D-gluconic acid) a compound that is known to be useful in medicine, but also in food products due to its sweet-sour taste. Lactobionic acid may be generated e.g. during manufacturing of milk products such as cheese and provide the resulting product with desired organoleptic properties and a reduced content of lactose. Furthermore, lactobionic acid *per se* and its salts may be generated e.g. by enzymatic conversion of lactose and used as additives in specific food products e.g. as an antioxidant, an emulsifying agent, as a general acidulent in food products, as a dietary mineral supplement and as a replacement of cheese starter culture in cheese manufacturing. In the pharmaceutical industry lactobionic acid is used e.g. as a key component in solutions for preservation of organs and in the cosmetic industry as an active ingredient in lotions and creams for general skin care. Additionally, lactobionic acid may be used in technical applications e.g. as an ingredient in detergents.

Lactobionic acid generated by the enzymatic conversion of lactose is preferred in most applications and carbohydrate oxidase enzymes capable of converting lactose to lactobionic acid are well known. The reaction scheme can be described by:

lactose + O₂ + H₂O→ lactobionic acid + H₂O₂

The CAS Reg. No. for lactobionic acid is 96-82-2.

WO02/089592 (Kraft Foods) describes a number of advantages of using lactobionic acid in milk based dairy products and advantages of using carbohydrate oxidase based enzymatic *in situ* conversion of lactose into lactobionic acid during the preparation of milk based dairy products such as cheese.

The advantages relate e.g. to preparation of dairy products with a reduced lactose content (e.g. milk reduced in lactose) and manufacturing of processed cheese products (e.g. for pizza) that have reduced problems with browning. Additionally, in e.g. cheese production lactobionic acid may be used to develop acidity. Generally, acidity is developed by fermenting milk with lactic acid bacteria that metabolize lactose to produce lactic acid. Consequently, by addition of lactobionic acid it is possible to produce relevant dairy products using reduced amounts of lactic acid bacteria.

With respect to the carbohydrate oxidase based enzymatic conversion of lactose into lactobionic acid, WO02/089592 describes that the enzyme is to be added to a dairy substrate (e.g. milk or whey) and then incubated for a certain period of time at a suitable temperature in order to accomplish the enzyme reaction. The description provides no explicit teaching with respect to any advantages of maintaining pH at a certain level during the enzymatic reaction. Actually, pH is allowed to decrease during the reaction, due to generation of lactobionic acid, and thereby acidifying the dairy product.

In example 9 of WO02/089592 milk is incubated with the carbohydrate oxidase overnight. No reference is made to any control of pH. Following overnight incubation, the treated milk comprised 1.5% lactose and 3.2% lactobionic acid, thus, providing a conversion of lactose to lactobionic acid of 68%.

In example 12 B) and C) milk is incubated with the carbohydrate oxidase for 48 hours at 55°C. During the reaction pH is maintained at pH 7. No explanation is given for this pH control and no lactobionic acid yields are provided in these examples.

WO03/037093 (Novozymes) also describes a carbohydrate oxidase based enzymatic conversion of lactose into lactobionic acid during the process of preparing milk based dairy products. As for WO02/089592, this document is also silent with respect to any advantages of maintaining the pH at a certain level during the enzymatic reaction. The only specific working example describes that full fat milk was incubated with the oxidase and allowed to react at 40°C until pH 4.2 was reached. It thus appears that pH was not controlled, as pH of natural fresh milk is approximately 6.6.

WO02/39828 (Danisco) relates to a process where a carbohydrate oxidase (hexose oxidase) solution is sprayed onto a pizza with cheese and the advantage of less browning (termed "Maillard reaction") of the pizza cheese is demonstrated. This document is also silent with respect to any advantages of maintaining the pH at a certain level during the enzymatic reaction.

Murakami (J. Appl. Glycosci. 50.117-120 (2003)) discloses a method for microbial production of lactobionic acid from lactose, using a mutated strain of *Burkholderia cepacia.*

Rand (Journal of Diary Science, 1144-50 (1975)) discloses an enzymatic process for acidifying milk. It is taught that lactose in the milk can be converted to gluconic acid by a coupled enzymatic reaction using the enzymes lactase and glucose oxidase.

Thus, in the art several documents disclose food-manufacturing processes involving enzymatic conversion of lactose to lactobionic acid. However, improved enzymatic processes useful for the above described purposes and particularly for industrial production of lactobionic acid *per se* are needed.

### SUMMARY OF THE INVENTION:

The problem to be solved by the present invention is to provide a process of obtaining increased yields and/or a reduced reaction time in enzymatic conversion of lactose to lactobionic acid from a dairy substrate. The yield is defined as the fraction of lactose converted to lactobionic acid at a given time. Thus, in the present context yield is equivalent to "degree of conversion" at a given time. In situations where a specific degree of conversion is wanted the present invention provides a process resulting in a reduced reaction time.

The present invention is based on the surprising observation that by maintaining pH at a stable value during the carbohydrate oxidase based enzymatic conversion of lactose into lactobionic acid, higher yield and/or a reduced reaction time in the enzymatic conversion could be achieved.

Accordingly, a first aspect of the invention relates to a process of obtaining increased yield and/or a reduced reaction time in enzymatic conversion of lactose to lactobionic acid, said process comprising:
i) adding to a dairy substrate a carbohydrate oxidase,
ii) incubating said dairy substrate under conditions allowing the carbohydrate oxidase to convert lactose to lactobionic acid,
iii) maintaining pH during incubation in the range of 3.0 to 9.0 by addition of a weak base, i.e a base having a pKb-value of at least 3.5, and thereby obtaining said increased yield and/or reduced reaction time.

A further preferred embodiment comprises the addition of a catalase optionally in combination with H₂O₂ in step i) of the process.

A further important aspect of the present invention is a process of obtaining increased yield and/or a reduced reaction time in enzymatic conversion of lactose to lactobionic acid, said process comprising:
i) adding to a dairy substrate a carbohydrate oxidase and a catalase,
ii) incubating said dairy substrate under conditions allowing the carbohydrate oxidase to convert lactose to lactobionic acid,
iii) maintaining pH during incubation in the range of 3.0 to 9.0 by addition of a base, wherein
   a. the base is Ca(OH)2, KOH, Mg(OH)2 or a weak base, i.e. a base having a pKb-value of at least 3.5, provided that when a strong base is used said pH is not a pH of 7.0, and/or
   b. the pH is maintained at a pH from 3.0 to 6.9 or 7.1 to 9.0, and thereby obtain said increased yield and/or reduced reaction time.

The enzymatic conversion of lactose to lactobionic acid requires oxygen and H₂O₂ is produced during the conversion. Addition of a catalase converts the produced H₂O₂ to oxygen. Thus, oxygen supply may be reduced when a catalase is introduced in the process.

In a still further aspect the invention provides a process according to any of the above aspects as an integrated part of a food manufacturing process.

### DETAILED DESCRIPTION OF THE INVENTION:

### Dairy substrate:

The term "dairy substrate" is to be understood as a solution/suspension of any milk or milk like product including lactose, such as whole or low fat milk, skim milk, buttermilk, condensed milk, dried milk, whey, whey permeate, lactose, mother liquid from crystallization of lactose, whey protein concentrate, or cream originating from any animal. The lactose present in the substrate does not necessarily have to be fully soluble, i.e. the reaction may be carried out e.g. in a concentrated slurry with a lactose content exceeding the content soluble at the reaction temperature. Furthermore, the reaction may be carried out in a dairy substrate where part of the lactose is or has been hydrolysed by lactase. In this case not only lactobionic acid is produced, but also galac-turonic acid and gluconic acid will be generated by the action of the carbohydrate oxidase.

Preferably, the dairy substrate is milk and more preferably whey or fractions of whey or a lactose solution/suspension.

The term "Milk" is to be understood as the lacteal secretion obtained by milking any mammal, such as cows, sheep, goats, buffaloes or camels.

As will be further described hereinafter, the present process is especially suitable for relatively large-scale production of lactobionic acid. Accordingly, in a preferred embodiment of the present invention the dairy substrate is used in an amount from 50 kg to 500000 kg.

### Lactobionic acid:

It is to be understood that the term "lactobionic acid" relates to lactobionic acid or salts thereof. Suitable salts include Na-lactobionate, Ca-lactobionate, NH₄-lactobionate and K- lactobionate.

The base used for pH control is selected to control the type of lactobionate produced. From lactobionate produced by controlling pH purified lactobionic acid may be obtained by elimination of the cations e.g. using ion exchange. In addition, cations such as Calcium may be eliminated using sedimentation with a strong acid, e.g. sulphuric acid or phosphoric acid.

### Carbohydrate oxidase:

A number of suitable carbohydrate oxidases, capable of converting lactose to lactobionic acid, are known and available to the skilled person. It may for instance be a hexose oxidase or a glucose oxidase.

A presently preferred carbohydrate oxidase is a microbial carbohydrate oxidase.

A suitable hexose oxidase (EC 1.1.3.5) is described in WO96/40935 (Bioteknologisk Institut, Denmark). This document describes a suitable hexose oxidase from marine algal species, more particular wherein the marine algal species is one selected from the group consisting of *Chon-drus crispus, Iridophycus flaccidum* and *Euthora cristata.*

Other suitable carbohydrate oxidases may be derived, e.g. from a mitosporic *Pyrenomycetes* such as *Acremonium,* in particular, *A. strictum,* such as ATCC 34717 or T1; *A. fusidioides,* such as IFO 6813; or *Apotronii,* such as IFO 31197. In a preferred embodiment, the carbohydrate oxidase is obtained from the source disclosed by Lin, et al, (1991, Biochim. Biophys. Acta 1118:41-47) and in JP-A 5-84074.

In a preferred embodiment the carbohydrate oxidase is a carbohydrate oxidase obtained from a fungus belonging to the genus *Microdochium,* more preferably wherein the fungus is *Microdochium nivale* and even more preferably wherein the fungus is *Microdochium nivale* CBS 100236. Such a preferred oxidase is described in details in WO99/31990 (Novo Nordisk A/S).

The amount of oxidase to be used will generally depend on the specific requirements and on the specific enzyme. The amount of oxidase addition preferably is sufficient to generate the desired degree of conversion of lactose to lactobionic acid within a specified time. Typically, an oxidase addition in the range from about 1 to about 10000 OXU per kg of dairy substrate is sufficient, particularly from about 5 to about 5000 OXU per kg of dairy substrate, and more particularly from about 5 to about 100 OXU per kg of dairy substrate. It is within the general knowledge of the skilled person to adjust the amount of specific enzyme needed for conversion of a specific dairy substrate.

In the literature an Oxidase Unit (OXU) is normally defined as the amount of enzyme that oxidizes one µmol lactose per minute under specific conditions. However, in the examples provide herein OXU is defined as one mg of pure lactose oxidase enzyme - as measured relative to an enzyme standard.

### Incubating under conditions allowing the carbohydrate oxidase to convert lactose to lactobionic acid:

The dairy substrate obtained in step i) of the present process is incubated under conditions allowing the carbohydrate oxidase to convert lactose to lactobionic acid. Such conditions include, but are not limited to, temperature, oxygen, amount and characteristics of carbohydrate oxidase, other additives such as e.g. catalase and reaction/incubation time. Obviously, incubation conditions are selected so as to support the achievement of the present invention, i.e. to obtain an increased yield and/or a reduced reaction time of enzymatic conversion of lactose to lactobionic acid.

Generally, a suitable incubation time should allow the degree of conversion of lactose to lactobionic acid of interest.

Oxygen is an important factor in the present process as the conversion of lactose to lactobionic acid consumes oxygen. This can e.g. be seen in the table of example 1 herein. Accordingly, if the oxygen is monitored during the enzymatic reaction one will generally observe an initial drop in the oxygen amount, which, if e.g. air is constantly provided, will return to around the initial level, when the enzyme reaction terminates. When oxygen has returned to more than 90% of the initial level it indicates that the enzymatic reaction has ended or at least been significantly slowed down. Accordingly, a suitable incubation time could preferably be a time that at least lasts until the oxygen level of the incubated dairy substrate has returned to more than 90% of the initial level. This is especially if a maximum conversion of lactose is desired.

As will be further described hereinafter in a specific aspect of the invention, it may be preferred to add a catalase during the enzymatic conversion of lactose. The catalase may be added at any suitable time e.g. in step i) of the process. Optionally, H₂O₂ is added in combination with the catalase.
Determining the amount of base equivalents added to keep pH constant can also be used to monitor the reaction.

Generally, a suitable incubation time is selected in the range from ½ hour to 3 days, most preferably from 2 hours to 48 hours.

The incubation temperature will generally depend on the carbohydrate oxidase used and is typically selected according to the optimal reaction temperature for the carbohydrate oxidase. However, as the solubility of oxygen decreases with increasing temperature, other factors have to be taken into account in order to obtain an optimal process. The skilled person will know how to balance the optimal temperature with respect to e.g. enzyme activity and oxygen solubility. Generally, a suitable temperature will be in the range from about 0°C to about 80°C. Low temperature, as for instance 5°C, results in a relatively slow reaction rate, but has the advantage of representing the typical temperature for cold stored products. Thus, it will be possible to run the reaction during manufacturing and/or storage of a dairy product.

Suitable sources of oxygen include atmospheric air, atmospheric air enriched in oxygen and pure oxygen. Running the process under a pressure higher than 1 atmosphere increases the solubility of oxygen and may be preferred wherever applicable.

The oxygen may be supplied to the process, e.g. by continuously mixing air into the dairy substrate during incubation. A further option for providing O₂ is by adding H₂O₂ in the presence of catalase. Use of H₂O₂ as an oxygen source may be particularly preferred when the process is carried out using immobilized enzymes where addition of oxygen is more difficult.

### Incubating at a stable pH:

As explained above, an essential feature of the present process as described herein is that during the incubation (step (ii)) the pH is to be maintained, by adequate addition of a base, at a stable level, provided that when a strong base is used said stable level is not a pH of 7.0. In specific embodiments the stable pH level is maintained in the range of from about 3.0 to about 9.0 by addition of a weak base or in the range from about 3.0 to 6.9 and from 7.1 to about 9.0 by addition of any base, such as in the range of from about 3.0 to 6.8 and from 7.2 to about 9.0, including a range from about 3.0 to 6.5 and from 7.5 to about 9.0, such as a range from about 3.0 to 6.0 and from 8.0 to about 9.0. Preferably, incubation is performed for a period of time that is sufficient to obtain a degree of conversion of lactose to lactobionic acid that is at least 2.5% higher than in a comparative control process where the only comparative difference is that during the incubation the pH is not maintained by adequate addition of a base.

The comparative control process should, except for the non-addition of base to maintain the pH, be performed identical (i.e. identical oxidase in identical amount, same dairy substrate, same incubation time, temperature and presence of oxygen etc.) to the process for improving yield and/or reducing reaction time in enzymatic conversion of lactose to lactobionic acid as described herein. This is in accordance with the normal understanding of a control as used herein, as the control is made to identify the positive effect of maintaining pH stable.

The preferred stable pH value for a specific process of interest will, as it will be appreciated by the skilled person, depend on a number of factors. For instance, if the dairy substrate is milk, the natural pH is known to be around 6.6 and it would be preferred to maintain the pH at a value around 6.6, such as a pH from 6.3 to 6.9. As mentioned earlier, the conventional enzymatic conversion of lactose to lactobionic acid is allowed to run without any pH control as the process is typically used for acidification of the end product. An adjustment of pH at the end of the process has been described in cases were pH drops below a desired level, e.g. in the manufacturing of low lactose milk. Thus, any previous adjustment of pH has been for the pure sake of matching pH with the end product and not for an optimisation of the process as such.

It will be appreciated that the pH of the lactobionic acid product or composition comprising lactobionic acid according to the present process can also be adjusted to a preferred pH level after or at the end of the enzymatic conversion performed, e.g. when 95% of the desired conversion of lactose has been achieved, the pH may be allowed to drop to a desired level. The drop in pH in combination with air sparging further allows CO₂ to escape from the dairy substrate. This is of particular interest when a carbonate is used for pH control during the reaction.

In the present context "a stable pH level" is to be broadly understood as the control and maintenance of pH during the process within a specific range, or close to/at a specific value by addition of a base. Control and adjustment/maintenance of pH during an enzymatic process is a standard procedure that can be carried out with a very high degree of accuracy. Thus, a stable pH may be a value maintained at a constant level with a variation of less than 0.1 pH unit of variation or even less than 0.05 pH unit of variation. It follows that an optimal range may be defined for a specific enzymatic process according to the present invention and that pH can be controlled and maintained with the described degree of accuracy within this range. In the process of the invention, a suitable specific pH range or specific pH value is selected in the range from about pH 3 to about pH 9. Subject to the limitation that the specific pH value is not 7.0 when a strong base is used for pH adjustment.

Preferably the pH is maintained, by adequate addition of a base, at a pH from 5.5 to 6.9, more preferably at a pH from 6.0 to 6.9.

It is preferred that pH is maintained at the stable pH level as described herein from the start of the enzymatic reaction. In other words, immediately after the oxidase is added to the dairy substrate the base is added to maintain the stable pH as described herein.

Particularly, if a maximum conversion of lactose is desired the pH is maintained at the stable level as described herein for a period of time that at least last until the oxygen level of the incubated dairy substrate has returned to more than 90% of the initial level.

Preferably, the pH is maintained at the stable pH level as described herein for a time period from 30 minutes to 48 hours, more preferably from 1 hour to 36 hours and even more preferably from 2 hours to 24 hours.

In the present process it is possible to maintain pH within the prescribed ranges using any base. In principle, any substance capable of neutralising the produced acid will be applicable in the process. However, for practical uses weak bases and carbonates are preferred. Examples of weak bases include, but are not limited to, CaCO₃, Na₂CO₃, K₂CO₃, (NH₄)₂CO₃ and NH₄OH. Presently preferred weak bases are NH₄OH and CaCO₃.

The skilled person knows numerous of other bases that can be applied in the process of the invention, e.g. strong bases such as Ca(OH)₂, KOH, NaOH and Mg(OH)₂.

When the base is e.g. Ca(OH)₂ or CaCO₃ it is possible by using the process as described herein, to produce Ca-lactobionate from a suitable dairy substrate. This product can then be used as e.g. a dairy product additive or an ingredient as it is described in the art.

Thus, as previously described, a preferred base will generally be a base that relates to the desired mineral composition of the final product. In specific embodiments it may be desired to enrich milk in calcium. In this case, a preferred base is Ca(OH)₂ as carbonates are not suitable due to a unwanted influence on taste. (Example 4).

The term "weak" vs "strong" base refers to the ability of the base to dissociate. In the present context a weak base is defined as a base having a pKb-value of at least 3.5 (for diprotic bases like CO₃²⁻ this pKb-value refers to the first step).

Preferably, during the incubating (step (ii)) the pH is maintained, by adequate addition of a base for a time period that is sufficient to obtain a degree of conversion of lactose to lactobionic acid that is at least 5% higher than in a comparative control process where the only difference is that during the incubation the pH is not maintained by addition of a base, more preferably at least 15% higher than in the comparative control process, even more preferably at least 30% higher than in the comparative control process and most preferably at least 45% higher than in the comparative control process.

For illustration, in working examples 1 and 2 herein the degree of conversion of lactose to lactobionic acid of the control was 41 % and by maintaining pH in the range from 5-6 the degree of conversion increased to 90%. Further, as illustrated in example 5, it was surprisingly found that the use of a weak base (e.g. 0.5M Na₂CO₃) reduced the reaction time by 24% as compared to the similar process where pH was maintained by addition of a strong base (1M NaOH). Thus, use of a weak base in lieu of a strong base reduces reaction time of the present process. The reaction time may be reduced by at least 5% such as at least 25%, at least 50%, or even at least 80%.

Additionally, as illustrated in Example 5, a residual enzyme activity close to 100% was found after the conversion process was finished. This opens op for possibilities of reusing the enzyme in a new batch, e.g. by recovering the enzymes by membrane filtration.

In a specific aspect of the invention the process for obtaining an increased yield and/or a reduced reaction time in enzymatic conversion of lactose to lactobionic acid is defined by the steps:
i) adding to a dairy substrate a carbohydrate oxidase,
ii) incubating said dairy substrate under conditions allowing the carbohydrate oxidase to convert lactose to lactobionic acid,
iii) maintaining pH during incubation in the range of 3.0 to 9.0 by addition of a weak base, i.e. a base having a pKb-value of at least 3.5, and thereby obtaining said increased yield and/or decreased reaction time.

Thus, as it appears from the above description and from the examples provided herein, the present invention is a valuable contribution in industrial conversion of lactose to lactobionic acid as the features defining the present process allows for an economical production of lactobionic acid. It will be appreciated that various alterations, modifications and adaptations may be based on the present disclosure and are intended to be within the scope and spirit of the present process.

Obviously, the disclosed process is useful for an industrial production of lactobionic acid *per se.* However, the process may also form part of a manufacturing process for production of a dairy product such as cheese, youghurt, milk etc.

### Purification of the lactobionic acid:

Optionally, it is possible to purify the lactobionic acid in any suitable way to obtain a lactobionic acid product or a composition comprising lactobionic acid with a desired degree of lactobionic acid purity.

The skilled person will know how to perform a purification and depending on the specific needs of interest a composition comprising at least 30% lactobionic acid, at least 90% lactobionic acid or even 95% or 99% lactobionic acid may be obtained.

Suitable methods for purification of the lactobionic acid include filtration, ion exchange, concentration and drying.

A composition comprising lactobionic acid may be used in the manufacturing of food products as e.g. a food additive or a food ingredient. Furthermore, the composition may be used in other applications as mentioned hereinbefore.

### Starter culture comprising lactic acid bacteria:

In general, extra elements or ingredients may be included in the process as described herein in agreement with specific needs. The person skilled in the art will be aware of numerous of such extra elements or ingredients needed.

In a preferred embodiment a starter culture comprising lactic acid bacteria is included in the process as described herein. The starter culture may be added to the diary substrate before or after the oxidase, and optionally a catalase, is added to the substrate. This will depend on the specific fermentation profile of interest.

The term "lactic acid bacteria" denotes herein a group of Gram-positive, non-sporing bacteria, which carry out a lactic acid fermentation of sugars.

Among others, it includes species of lactic acid bacteria belonging to genus *Lactobacillus,* such as *Lactobacillus helveticus, Lactobacillus delbruckii subsp. bulgaricus,* etc., lactic acid bacteria belonging to genus *Lactococcus,* such as *Lactococcus lactis,* lactic acid bacteria belonging to genus *Streptococcus,* such as *Streptococcus salivarius,* lactic acid bacteria belonging to genus *Leuconostoc,* such as *Leuconostoc lactis,* lactic acid bacteria belonging to genus *Bifidobacterium,* such as *Bifidobacterium longuni* or *Bifidobacterium, breve,* and lactic acid bacteria belonging to genus *Pediococcus.*

The lactic acid bacteria may be used as a mixture with other microorganisms, e.g. yeasts.

### Catalase:

A catalase is an enzyme that catalyses the reaction: 2 H₂O₂ → O₂ + 2H₂O. The EC number is EC 1.11.1.6.

As shown in working example 3 herein use of a catalase significantly improves the degree of conversion of lactose to lactobionate acid. Furthermore, use of a catalase decreases the amount of H₂O₂ produced during conversion of lactose to lactobionic acid.

Thus, in a preferred embodiment of the present invention a catalase is added to the process of the invention.

As described above, when the carbohydrate oxidase converts lactose to lactobionic acid H₂O₂ is generated. The reaction scheme may be described as:

lactose + O₂ + H₂O → lactobionic acid + H₂O₂

The catalase may be added after the carbohydrate oxidase has reacted to generate the lactobionic acid. However, preferably the catalase is added together with the carbohydrate oxidase in step (i) of the process. An advantage of adding a catalase together with the carbohydrate oxidase in step (i) of the process is that the oxygen requirement can be significantly reduced (up to 50%). Thus, supply of oxygen, e.g. in the form of air may be significantly reduced. Actually, if one is adding an adequate amount of catalase together with H₂O₂ it is not necessary to supply extra oxygen e.g. in the form of air. This extra-added H₂O₂ may originate from any commercial source.

Accordingly, a preferred embodiment is wherein essentially all the oxygen required in step (ii) of the process is obtained by extra addition of H₂O₂ and wherein the catalase generates the required oxygen by conversion of the available H₂O₂.

In the present context the expression "essentially all of the oxygen" is used to describe the oxygen supply needed for the enzymatic reaction to work adequately and in particular that it is not necessary to actively add extra oxygen during the incubation step, e.g. by continuously mixing air into the dairy substrate under incubation.
A preferred embodiment is wherein pH during the incubating is maintained by adequate addition of a base for a time period that is sufficiently long to obtain a degree of conversion of lactose to lactobionic acid that is at least 2.5% higher than in a comparative control process where the only difference is that during the incubation the pH is not maintained by adequate addition of a base.

In a preferred embodiment catalase in step (i) of the process is added in an amount that lowers the concentration of H₂O₂ as compared to a similar process with no catalase added.

Preferably, the catalase is added in an amount that also improves the degree of conversion of lactose to lactobionate acid.

A number of suitable catalases are known to the skilled person. For instance, the commercially available catalase Catazyme® from Novozymes A/S.

The amount of catalase added to the process as described herein will generally depend on the amount of H₂O₂ desired in the final product. Accordingly, depending on the particular dairy product of interest, especially if the dairy product is milk, the amount of catalase added to the process as described herein, is in an amount that is sufficient to obtain an at least 10% decrease in the amount of H₂O₂ as compared to a comparative control process where the only comparative difference is that catalase is not added.

More preferably, the amount of catalase added to the process as described herein, is an amount that is sufficient to obtain an at least 25% decrease in the amount of H₂O₂ as compared to a comparative control process where the only comparative difference is that catalase is not added, even more preferably the amount of catalase added to the process as described herein, is an amount that is sufficient to obtain an at least 75% decrease in the amount of H₂O₂ as compared to a comparative control process where the only comparative difference is that catalase is not added.

The comparative control process should, except for the non-addition of the catalase, be performed identically (same oxidase in same amount, same dairy substrate, same incubation time, temperature, presence of oxygen and addition of base, etc.) to the process for improving lactobionic acid as described herein. This is in accordance with the normal understanding of a control as used herein, because the control is made to identify the positive effect of the addition of catalase.

In a separate aspect of the invention relates to a process for obtaining increased yield and/or a reduced reaction time in enzymatic conversion of lactose to lactobionic acid comprising:
(i) adding to a dairy substrate a carbohydrate oxidase and a catalase,
(ii) incubating said dairy substrate under conditions allowing the carbohydrate oxidase to convert lactose to lactobionic acid,
(iii) maintaining pH during incubation in the range of 3.0 to 9.0 by addition of a base, wherein
   a. the base is Ca(OH)2, KOH, Mg(OH)2 or a weak base, i.e. a base having a pKb-value of at least 3.5, provided that when a strong base is used said pH is not a pH of 7.0, and/or
   b. the pH is maintained at a pH from 3.0 to 6.9 or 7.1 to 9.0,
   and thereby obtaining said increased yield and/or reduced reaction time.

All embodiments, as described herein, with respect to the process of the first aspect of the present invention are also preferred embodiments with respect to the process of this separate aspect of the invention.

### A process for making a dairy product

As described above, the process according to any aspect of the invention may be an integrated part of any other process e.g. a food manufacturing process, such as a process for manufacturing of a dairy product, wherein conversion of lactose to lactobionic acid is desired. Thus, a final food product may be obtained using the present processes. Furthermore, a composition comprising lactobionic acid generated according to the processes of the present invention may be used in the manufacturing of e.g. a dairy product. The person of skill in the art will know of the relevant uses of lactobionic acid as such and a composition comprising lactobionic acid and reference is e.g. made to the prior art publications discussed above and the application mentioned in the background of the invention. Accordingly, once a composition comprising lactobionic acid or a lactobionic acid product *per se* is obtained by the present process the composition or product may be used in any suitable way.

The term "dairy product" is to be understood as any dairy product including a dairy substrate, as defined above. Examples of dairy products applicable for the present invention are products like yoghurt, milk such as e.g. a calcium fortified milk and cheese such as process cheese (e.g. for pizza), cream cheese and cottage cheese.

All embodiments, as described herein, with respect to the process of the previous aspects of the present invention are also preferred embodiments with respect to the process of this aspect of the invention.

Having generally described the aspects and embodiments of the present process, the invention will now be described using specific examples. The examples further illustrate various features and advantages of the invention, but are not intended to limit the scope of the invention.

### EXAMPLES:

### Example 1: (Comparative)

Experiments were performed in a large scale trial with 400 kg substrate solution consisting of 6% whey permeate powder (from EPI, France). Temperature was adjusted to 49°C.
A carbohydrate oxidase enzyme dosage corresponding to 40000 OXU in 400 litre solution = 1250 g enzyme solution was used. The enzyme was achieved from *Microdochium nivale* as described in patent application WO 9931990. In the literature, an Oxidase Unit (OXU) is normally defined as the amount of enzyme that oxidizes one µmol lactose per minute under a specific set of conditions. In the present examples, however, one OXU is defined as one mg of pure lactose oxidase enzyme - measured relative to an enzyme standard.

The reaction was followed by monitoring pH and the oxygen tension of the solution. Air was mixed into the substrate by re-circulation of substrate to the tank utilizing the pump turbulence (Landia pump 5.5kWh).
When oxygen returned to >90% the reaction is finished and the product was heated to 85°C for enzyme inactivation. Samples were taken during the reaction, and were heated to 85°C for inactivation of the enzyme.
pH in substrate solution before enzyme addition was 6.52. After termination (5 hours reaction time) of the reaction pH had decreased to 3.62. Oxygen content in substrate was 5.82mg/L at time = 0 and reduced within 5 minutes after enzyme addition to less than 0.5mg/L. Data from the trial is seen in the table below.

| **Time, minutes** | **pH** | **Oxygen** |
|---|---|---|
| 0 | 6,52 | 5,82 |
| 5 | 6,39 | 0,50 |
| 15 | 6,02 | 0,49 |
| 30 | 5,63 | 0,33 |
| 60 | 5,12 | 0,40 |
| 65 | 5,02 | 0,40 |
| 90 | 4,82 | 0,42 |
| 120 | 4,46 | 0,32 |
| 175 | 4,00 | 0,12 |
| 240 | 3,70 | 0,90 |
| 250 | 3,66 | 1,70 |
| 260 | 3,64 | 3,66 |
| 270 | 3,64 | 4,00 |
| 280 | 3,64 | 4,45 |
| 290 | 3,62 | 5,45 |
| 300 | 3,62 | 5,52 |

Oxygen content is mg/L

### Degree of conversion of lactose to lactobionic acid was 41%.

### Example 2 (not pertaining to the invention)

833g (corresponding to 26700 OXU) carbohydrare oxidase was added to 166 kg substrate similar to the one described in example 1. The oxidase used was the same as in example 1. pH was held in the range 5 - 6 by addition of 5N NaOH solution. When the oxygen level returned to the initial level (after 5 hours) a total amount of 3910 mL 5N NaOH had been used for pH-control. This amount corresponds to a conversion of > 90% lactose to lactobionate according to the reaction scheme:

lactose + O₂ + H₂O → lactobionic acid + H₂O₂, where the NaOH is balance the acid formed.

### Example 3

441g (corresponding to 14100 OXU) carbohydrate oxidase solution and 39g catalase 25L was added to 166 kg substrate similar to the one described in example 1. The oxidase used was the same as in example 1. The Catalase was Catazyme® from Novozymes A/S.

pH was held in the range 5 - 6 by addition of 5N NaOH solution. When the oxygen level returned to the initial level (after 4 hours 40 minutes) a total amount of 4860g 5N NaOH had been used for pH-control. This amount corresponds to a conversion of 100% lactose to lactobionate acid.

### Example 4:

### Objective

The objective was to test the concept of producing Ca-fortified milk by lactose oxidase catalysed reaction in milk by keeping pH constant by Ca(OH)2 addition. Samples were taken at different base additions and the milk was heat treated and evaluated (taste and stability).

### Method

Substrate skim milk 1.5kg
Temperature 50C
Fresh air was flushed over the surface of the stirred substrate.
The enzyme was a carbohydrate oxidase preparation from *M.nivale* dosage 0.0850XU/g solution = 3.98g enzyme solution. The oxidase used was the same as in example 1.
Catalase 25L dosage 0.5g (Catazyme® from Novozymes A/S).
The oxidase and catalase was added to the substrate and incubated as described below.
pH was kept constant by addition of 1N Ca(OH)₂
Samples of 100mL were taken after base consumption of 22.2mL and 41.15mL, and heat treated at 85°C for 15 minutes.
After cooling the samples were evaluated with respect to taste and stability.

### Results

pH in skim milk is 6.65 at 50°C, which was used as the set-point in the pH-stat titration unit. pH in the samples were 6.76 and 6.88 measured at room temperature.
None of the samples were grainy or had any sign of sediment. The last sample seemed slightly more viscous. Taste was good for both samples and no off flavour was detected.

The Ca-level in the milk after the Ca(OH)₂ addition had increased:
Sample 1:48%
Sample 2: 88%

The degree of conversion of lactose to LBA estimated from the amount of base used was:
Sample 1: 22.2mL corresponds to 11% conversion
Sample 2: 41.15mL corresponds to 20% conversion

### Conclusion

This example demonstrates that a Ca-fortified milk without taste defects can be produced by addition of Ca(OH)₂ during enzymatic oxidation of lactose to lactobionic acid as long as the addition of base is done in a titration equipment keeping pH constant. Ca-enrichment up to 88% in the skim milk was obtained. This level by far exceeds the level normally aimed at in milk drinks.

### Example 5:

### Objective

The objective was to test the effect of using different bases for pH-regulation during the lactose oxidase catalysed oxidation of lactose to lactobionic acid

### Method

The experiments were performed in a lab-scale bio-reactor with a working volume of 1.0 L. The reactor is equipped with two Rushton turbines working at 1,000 rotations per minute (RPM). Dissolved oxygen was measured by a Mettler-Toledo polarographic sensor, and controlled at 44.1 % (relative to saturation with air at 38°C) by feed-back control of two mass flow controllers dispensing respectively nitrogen and air. The total flow rate was set to 200 mL/min.
Substrate: 1.0 L aqueous 50 g/L lactose with 50 mM HPO₄²⁻/H₂PO₄⁻ buffer
Temperature: 38°C
pH: Measured and kept constant at 6.40 by addition of base

The enzyme used was a carbohydrate oxidase preparation from *M. nivale* dosage 60 OXU/L solution. The catalase 25L dosage was 0.25 g (Catazyme® from Novozymes A/S). The rate of reaction and thus the evolution of the reaction were followed by monitoring the amount of base used for pH-regulation

### Results

Five different bases were used for neutralization in experiments which were identical in all other aspects. In all 5 cases the conversion of lactose (calculated by base addition) was 100%. However, the total reaction time was dependent on the alkalinity of the base used, see the table below. When using a strong base like NaOH the reaction time was longer than if a weak base like NH₃ or CO₃²⁻ was used. The rate of reaction declined with time when NaOH was used, while this was not the case when NH₃ or CO₃²⁻ was used (before lactose was depleted). This is reflected in the residual activity relative to that at time = 0 which is given in the table.

| | Time for completion (h) | Residual oxidase activity (%) |
|---|---|---|
| 2.0 M NaOH | 6.33 | 50 |
| 1.0 M NaOH | 6.17 | 52 |
| 0.5 M NaOH | 6.00 | 62 |
| 0.5 M Na₂CO₃ | 4.66 | >95 |
| 1.8 M NH₃ | 4.33 | >95 |

### Conclusion

This example demonstrates that it is highly advantageous to use a weak base for neutralization.

## Claims

1. A process of obtaining increased yield and/or a reduced reaction time in enzymatic conversion of lactose to lactobionic acid, said process comprising:
i) adding to a dairy substrate a carbohydrate oxidase,
ii) incubating said dairy substrate under conditions allowing the carbohydrate oxidase to convert lactose to lactobionic acid,
iii) maintaining pH during incubation in the range of 3.0 to 9.0 by addition of a weak base, i.e. a base having a pKb-value of at least 3.5, and thereby obtaining said increased yield and/or reduced reaction time.

2. A process of obtaining increased yield and/or a reduced reaction time in enzymatic conversion of lactose to lactobionic acid, said process comprising:
i) adding to a dairy substrate a carbohydrate oxidase and a catalase,
ii) incubating said dairy substrate under conditions allowing the carbohydrate oxidase to convert lactose to lactobionic acid,
iii) maintaining pH during incubation in the range of 3.0 to 9.0 by addition of a base, wherein
a. the base is Ca(OH)₂, KOH, Mg(OH)₂ or a weak base, i.e. a base having a pKb-value of at least 3.5, provided that when a strong base is used said pH is not a pH of 7.0, and/or
b. the pH is maintained at a pH from 3.0 to 6.9 or 7.1 to 9.0,
and thereby obtain said increased yield and/or reduced reaction time.

3. The process of any of the preceding claims, wherein a catalase is added in step (i) of the process in an amount that decreases the amount of H₂O₂ produced during conversion of lactose, such as inan amount sufficient to obtain an at least 10% decrease in the concentration of H₂O₂ as compared to a control process where the only comparative difference is that catalase is not added.

4. The process of claims 3, wherein essentially all of the suitable amount of oxygen required in step (ii) is obtained by extra addition of a suitable amount of H₂O₂ and wherein the catalase generates the required oxygen from the available H₂O₂.

5. The process according to any of the preceding claims, wherein the base is a weak base, i.e a base having a pKb-value of at least 3.5, such as Na₂CO₃ or NH₄OH

6. The process according to any of the preceding claims, wherein the dairy substrate is milk, whey or fractions of whey or a lactose solution/suspension.

7. The process according to any the preceding claims, wherein the carbohydrate oxidase is a microbial carbohydrate oxidase, such as a carbohydrate oxidase is a carbohydrate oxidase obtained from a fungus belonging to the genus *Microdochium,* more preferably wherein the fungus is *Microdochium nivale* and even more preferably wherein the fungus is *Microdochium nivale* CBS 100236.

8. The process of any of the preceding claims, wherein the pH is maintained at the stable pH level as described herein for a time period from 30 minutes to 48 hours, more preferably from 1 hour to 36 hours and even more preferably from 2 hours to 24 hours.

9. The process of any of the preceding claims, wherein a starter culture comprising lactic acid bacteria is included in the process and wherein the starter culture may be added to the diary substrate before or after the oxidase is added.

10. A process according to any of the preceding claims as an integrated part of a food manufacturing process, such as a process for manufacturing of a dairy product such as a yoghurt, a milk such as e.g. a calcium fortified milk and a cheese such as process cheese (e.g. for pizza), cream cheese and cottage cheese.

11. The process according to any of the preceding claims, wherein the pH is maintained at a pH from 3.0 to 9.0, such as at a pH from 5.5 to 6.9 or at a pH from 6.0 to 9.0.

## Patentansprüche

1. Verfahren zur Erzielung einer erhöhten Ausbeute und/oder einer verkürzten Reaktionszeit bei der enzymatischen Umwandlung von Lactose in Lactobionsäure, wobei das Verfahren Folgendes umfasst:
(i) Versetzen eines Milchsubstrats mit einer Kohlenhydrat-Oxidase,
(ii) Inkubieren des Milchsubstrats unter Bedingungen, die der Kohlenhydrat-Oxidase das Umwandeln von Lactose in Lactobionsäure gestatten,
(iii) Halten des pH-Werts während der Inkubation im Bereich von 3,0 bis 9,0 durch Zugabe einer schwachen Base, d.h. einer Base mit einem pKb-Wert von wenigstens 3,5, und damit Erzielen der erhöhten Ausbeute und/oder verkürzten Reaktionszeit.

2. Verfahren zur Erzielung einer erhöhten Ausbeute und/oder einer verkürzten Reaktionszeit bei der enzymatischen Umwandlung von Lactose in Lactobionsäure, wobei das Verfahren Folgendes umfasst:
(i) Versetzen eines Milchsubstrats mit einer Kohlenhydrat-Oxidase und einer Katalase,
(ii) Inkubieren des Milchsubstrats unter Bedingungen, die der Kohlenhydrat-Oxidase das Umwandeln von Lactose in Lactobionsäure gestatten,
(iii) Halten des pH-Werts während der Inkubation im Bereich von 3,0 bis 9,0 durch Zugabe einer Base, wobei
a. es sich bei der Base um Ca(OH)₂, KOH, Mg(OH)₂ oder eine schwache Base, d.h. eine Base mit einem pKb-Wert von wenigstens 3,5, handelt, vorausgesetzt, dass, wenn eine starke Base verwendet wird, der pH nicht bei einem pH-Wert von 7,0 liegt, und/oder
b. der pH bei einem pH-Wert von 3,0 bis 6,9 oder 7,1 bis 9,0 gehalten wird,
und damit Erzielen der erhöhten Ausbeute und/oder verkürzten Reaktionszeit.

3. Verfahren nach einem der vorhergehenden Ansprüche, wobei in Schritt (i) des Verfahrens eine Katalase in einer Menge zugegeben wird, durch die die Menge an während der Umwandlung von Lactose produziertem H₂O₂ verringert wird, wie etwa einer hinreichenden Menge, so dass man eine wenigstens 10%ige Verringerung der H₂O₂-Konzentration verglichen mit einem Kontrollverfahren erhält, bei dem der einzige Vergleichsunterschied darin besteht, dass Katalase nicht zugegeben wird.

4. Verfahren nach Anspruch 3, wobei im Wesentlichen die gesamte in Schritt (ii) erforderliche geeignete Menge an Sauerstoff durch eine zusätzliche Zugabe einer geeigneten Menge an H₂O₂ erhalten wird und wobei die Katalase den erforderlichen Sauerstoff aus dem verfügbaren H₂O₂ erzeugt.

5. Verfahren gemäß einem der vorhergehenden Ansprüche, wobei es sich bei der Base um eine schwache Base, d.h. eine Base mit einem pKb-Wert von wenigstens 3,5, handelt, wie etwa Na₂CO₃ oder NH₄OH.

6. Verfahren gemäß einem der vorhergehenden Ansprüche, wobei es sich bei dem Milchsubstrat um Milch, Molke oder Molkefraktionen oder eine Lactoselösung bzw. -suspension handelt.

7. Verfahren gemäß einem der vorhergehenden Ansprüche, wobei es sich bei der Kohlenhydrat-Oxidase um eine mikrobielle Kohlenhydrat-Oxidase handelt, es sich z.B. bei der Kohlenhydrat-Oxidase um eine Kohlenhydrat-Oxidase handelt, die aus einem zur Gattung *Microdochium* gehörenden Pilz erhalten wird, stärker bevorzugt, wobei es sich bei dem Pilz um *Microdochium nivale* handelt, und noch stärker bevorzugt, wobei es sich bei dem Pilz um *Microdochium nivale* CBS 100236 handelt.

8. Verfahren nach einem der vorhergehenden Ansprüche, wobei der pH auf dem stabilen pH-Niveau wie vorliegend beschrieben über einen Zeitraum von 30 Minuten bis 48 Stunden, stärker bevorzugt von 1 Stunde bis 36 Stunden und noch stärker bevorzugt von 2 Stunden bis 24 Stunden gehalten wird.

9. Verfahren nach einem der vorhergehenden Ansprüche, wobei eine Milchsäurebakterien umfassende Starterkultur im Verfahren enthalten ist und wobei die Starterkultur vor oder nach Zugabe der Oxidase zum Milchsubstrat gegeben werden kann.

10. Verfahren gemäß einem der vorhergehenden Ansprüche als integrierter Teil eines Lebensmittelherstellungsverfahrens, wie etwa eines Verfahrens zur Herstellung eines Milcherzeugnisses, wie etwa eines Joghurts, einer Milch, wie z.B. einer mit Calcium angereicherten Milch, und eines Käses, wie etwa Schmelzkäses (z.B. für Pizza), Frischkäses und Hüttenkäses.

11. Verfahren gemäß einem der vorhergehenden Ansprüche, wobei der pH bei einem pH-Wert von 3,0 bis 9,0, wie etwa bei einem pH-Wert von 5,5 bis 6,9 oder bei einem pH-Wert von 6,0 bis 9,0, gehalten wird.

## Revendications

1. Procédé d'obtention d'un rendement accru et/ou d'un temps de réaction réduit dans la conversion enzymatique du lactose en acide lactobionique, ledit procédé comprenant :
i) l'addition, à un substrat laitier, d'une glucide oxydase,
ii) l'incubation dudit substrat laitier dans des conditions permettant à la glucide oxydase de convertir le lactose en acide lactobionique,
iii) le maintien du pH pendant l'incubation dans la plage allant de 3,0 à 9,0 par l'addition d'une base faible, c'est-à-dire une base ayant une valeur de pK_{b} d'au moins 3,5, pour obtenir ainsi ledit rendement accru et/ou temps de réaction réduit.

2. Procédé d'obtention d'un rendement accru et/ou d'un temps de réaction réduit dans la conversion enzymatique du lactose en acide lactobionique, ledit procédé comprenant :
i) l'addition, à un substrat laitier, d'une glucide oxydase et d'une catalase,
ii) l'incubation dudit substrat laitier dans des conditions permettant à la glucide oxydase de convertir le lactose en acide lactobionique,
iii) le maintien du pH pendant l'incubation dans la plage allant de 3,0 à 9,0 par l'addition d'une base, où a. la base est Ca(OH)₂, KOH, Mg(OH)₂ ou une base faible, c'est-à-dire une base ayant une valeur de pK_{b} d'au moins 3,5, à condition que lorsqu'une base forte est utilisée, ledit pH ne soit pas un pH de 7,0, et/ou b. le pH est maintenu à un pH allant de 3,0 à 6,9 ou de 7,1 à 9,0,
pour obtenir ainsi ledit rendement accru et/ou temps de réaction réduit.

3. Procédé selon l'une quelconque des revendications précédentes, dans lequel une catalase est ajoutée dans l'étape (i) du procédé selon une quantité qui réduit la quantité de H₂O₂ produite lors de la conversion du lactose, tel que selon une quantité suffisante pour obtenir une réduction d'au moins 10% de la concentration en H₂O₂ par rapport à un procédé témoin où la seule différence comparative est que la catalase n'est pas ajoutée.

4. Procédé selon la revendication 3, dans lequel sensiblement l'ensemble de la quantité convenable en oxygène requise dans l'étape (ii) est obtenu par l'addition supplémentaire d'une quantité convenable de H₂O₂ et où la catalase génère l'oxygène requis à partir du H₂O₂ disponible.

5. Procédé selon l'une quelconque des revendications précédentes, dans lequel la base est une base faible, c'est-à-dire une base ayant une valeur de pK_{b} d'au moins 3,5, telle que Na₂CO₃ ou NH₄OH.

6. Procédé selon l'une quelconque des revendications précédentes, dans lequel le substrat laitier est du lait, du lactosérum ou des fractions de lactosérum ou une solution/suspension de lactose.

7. Procédé selon l'une quelconque des revendications précédentes, dans lequel la glucide oxydase est une glucide oxydase microbienne, tel que la glucide oxydase est une glucide oxydase obtenue à partir d'un champignon appartenant au genre *Microdochium,* plus préférablement où le champignon est *Microdochium nivale* et encore plus préférablement où le champignon est *Microdochium nivale* CBS 100236.

8. Procédé selon l'une quelconque des revendications précédentes, dans lequel le pH est maintenu au taux de pH stable tel que décrit ici pour une période de temps allant de 30 minutes à 48 heures, plus préférablement allant de 1 heure à 36 heures et encore plus préférablement allant de 2 heures à 24 heures.

9. Procédé selon l'une quelconque des revendications précédentes, dans lequel une culture starter comprenant des bactéries lactiques est incluse dans le procédé et où la culture starter peut être ajoutée au substrat laitier avant ou après l'addition de l'oxydase.

10. Procédé selon l'une quelconque des revendications précédentes, comme partie intégrée d'un procédé de fabrication alimentaire, tel qu'un procédé de fabrication d'un produit laitier tel qu'un yaourt, un lait tel que par exemple un lait fortifié par du calcium et un fromage tel qu'un fromage fondu (par exemple pour des pizzas), un fromage à la crème et un fromage cottage.

11. Procédé selon l'une quelconque des revendications précédentes, dans lequel le pH est maintenu à un pH allant de 3,0 à 9,0, tel qu'à un pH allant de 5,5 à 6,9 ou à un pH allant de 6,0 à 9,0.
